(19) Europäisches Patentamt
European Patent Office
Office européen des brevets

(11) Numéro de publication : **0 343 050 B1**

(12) # FASCICULE DE BREVET EUROPEEN

(45) Date de publication du fascicule du brevet : **02.12.92 Bulletin 92/49**

(51) Int. Cl.[5] : **C07D 239/54,** C07D 401/12, C07D 217/22, A61K 31/505

(21) Numéro de dépôt : **89401330.9**

(22) Date de dépôt : **12.05.89**

(54) **Dérivés de phényl-6 pipérazinyl-alkyl-3 1H,3H-pyrimidinedione-2,4, leur préparation et leur application en thérapeutique.**

Le dossier contient des informations techniques présentées postérieurement au dépôt de la demande et ne figurant pas dans le présent fascicule.

(30) Priorité : **17.05.88 FR 8806568**

(43) Date de publication de la demande : **23.11.89 Bulletin 89/47**

(45) Mention de la délivrance du brevet : **02.12.92 Bulletin 92/49**

(84) Etats contractants désignés : **AT BE CH DE ES FR GB GR IT LI LU NL SE**

(56) Documents cités :
**EP-A- 0 281 309**
**US-A- 4 625 028**
**JOURNAL OF THE ORGANIC CHEMICAL SOCIETY, vol. C, no. 10, 1971, pages 1945-1948, Londres, GB; J. CLARK et al.: "Heterocyclicstudies. Part XIX. Some 6-(substituted phenyl)-uracil and -thiouracil derivatives"**
**SYNTHESIS - JOURNAL OF SYNTHETIC ORGANIC CHEMISTRY, no. 1, janvier 1988, pages 70-72, Thieme Medical Publishers, Inc., NewYork, US; L. STREKOWSKI et al.: "Facile preparation of 6-substituted uracils"**

(56) Documents cités :
**CHEMICAL ABSTRACTS, vol. 106, 1987, page 650, résumé no. 102199w, Columbus, Ohio, US; M.M. AL-ARAB: "Reactions ofarylpropiolamides with arylacetamides"**
**J. MED. CHEM., vol. 29, no. 11, 1986, pages 2375-2380**

(73) Titulaire : **SYNTHELABO**
**22, Avenue Galilée**
**F-92350 Le Plessis Robinson (FR)**

(72) Inventeur : **Frost, Jonathan**
**23, Route de Montjean**
**F-91320 Wissous (FR)**
Inventeur : **Gaudilliere, Bernard**
**28, rue Zilina**
**F-92000 Nanterre (FR)**
Inventeur : **Rousseau, Jean**
**17Bis, Avenue de Montrouge**
**F-92340 Bourg la Reine (FR)**
Inventeur : **Dupont, Régis**
**152, Quai Paul Bert**
**F-37100 Tours (FR)**
Inventeur : **Manoury, Philippe**
**38, Avenue de Vaupépins**
**F-91370 Verrieres le Buisson (FR)**
Inventeur : **Obitz, Daniel**
**91, rue Boucicaut**
**F-92250 Fontenay aux Roses (FR)**

(74) Mandataire : **Ludwig, Jacques et al**
**SYNTHELABO Service Brevets 22, avenue Galilée**
**F-92352 LE PLESSIS ROBINSON CEDEX (FR)**

EP 0 343 050 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention a pour objet des dérivés de phényl-6 pipérazinylalkyl-3 1*H*,3*H*-pyrimidinedione-2,4, leur préparation et leur application en thérapeutique.

Les composés selon l'invention répondent à la formule générale (I) donnée dans le schéma de la page suivante, formule dans laquelle

R1 représente un atome d'hydrogène ou d'halogène ou un groupe méthyle ou méthoxy,

R2 représente un atome d'hydrogène, un groupe alkyle en $C_1$-$C_4$, ou un groupe benzyle,

n représente un nombre entier égal à 2, 3 ou 4, et

X représente un groupe CH ou un atome d'azote, et

R3 représente un atome d'hydrogène ou d'halogène ou un groupe méthoxy lorsque X représente un groupe CH, ou seulement un atome d'hydrogène lorsque X représente un atome d'azote.

Ils peuvent se présenter à l'état de bases libres ou de sels d'addition à des acides.

Des composés de structure proche de celle des composés de l'invention sont décrits dans J. Med. Chem., **29**(11), 2379 (1986) et dans la demande de brevet EP-281309.

Conformément à l'invention, on peut préparer les composés de formule générale (I) par un procédé illustré par le schéma 1 ci-après.

Le procédé consiste à faire réagir une phényl-6 1*H*,3*H*-pyrimidinedione-2,4 de formule générale (II) (dans laquelle R1 et R2 sont tels que définis ci-dessus) avec un dérivé dihalogéné de formule générale (III) (dans laquelle n est tel que défini ci-dessus et Y représente un atome de brome ou de chlore). La réaction s'effectue par exemple dans un solvant inerte, tel que le méthanol ou l'éthanol, ou bien sans autre solvant que le dérivé dihalogéné lui-même et en présence d'un agent de transfert de phase, par exemple le chlorure de triéthylbenzylammonium, en présence d'une base, par exemple la potasse, à la température du reflux.

Ensuite on fait réagir le dérivé de formule générale (IV) ainsi obtenu avec un dérivé de pipérazine de formule générale (V) (dans laquelle X est tel que défini ci-dessus).

La réaction s'effectue par exemple dans un solvant inerte, tel que le méthanol ou l'éthanol, à la température du reflux,

Schéma 1

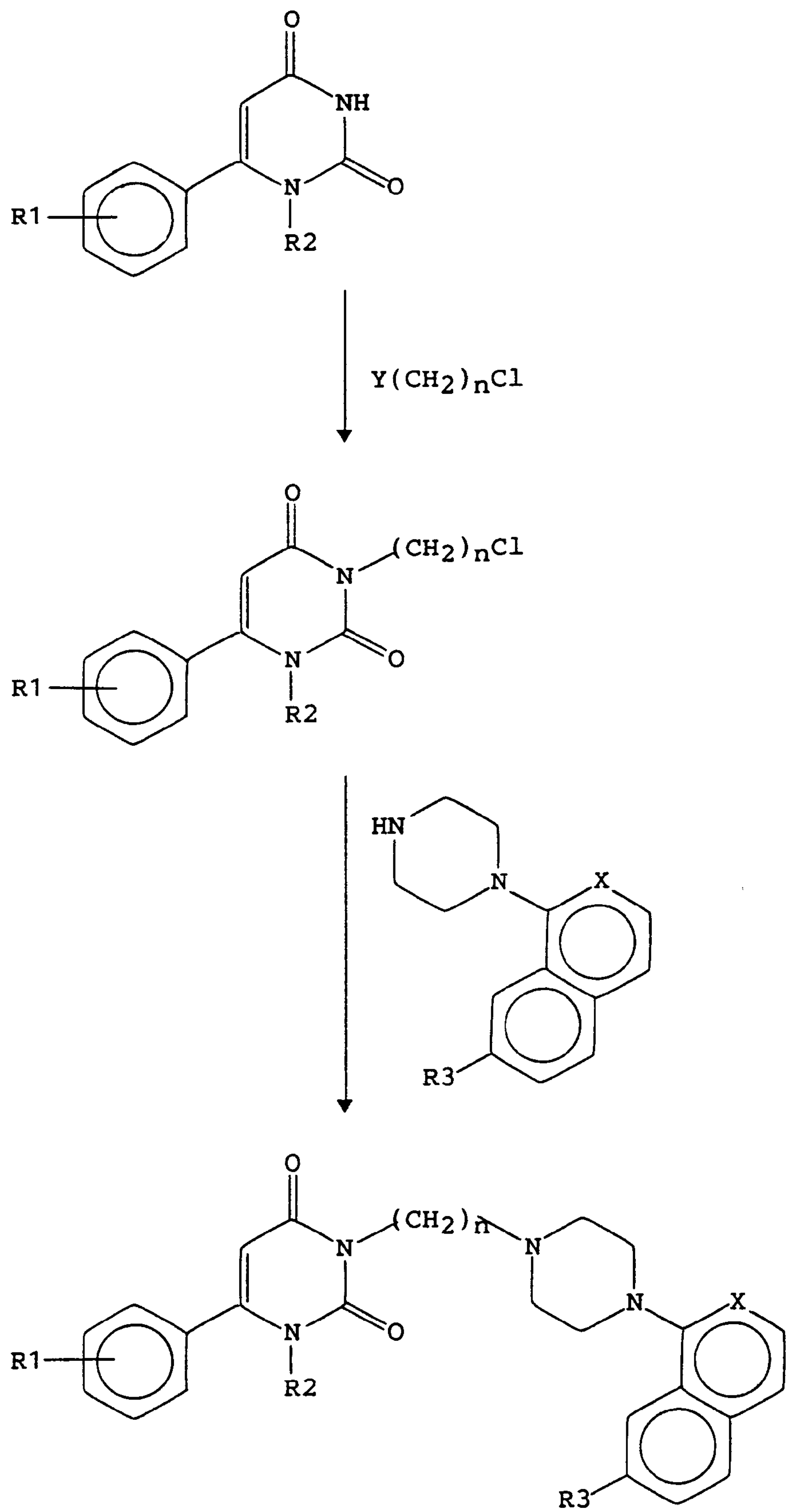


(II)

(III)

(IV)

(V)

(I)

ou bien sans solvant, à une température de 100 à 120°C, en présence d'une base, par exemple un excès de

dérivé de pipérazine de formule générale (V).

Lorsque R2 représente un atome d'hydrogène, on peut avantageusement mettre à profit l'énolisation de la liaison 1-2 du cycle de la pyrimidine de formule générale (II), selon une variante de procédé illustrée par le schéma 2 ci-après.

On traite d'abord un composé de formule générale (IIa, IIb) (dans laquelle R1 est tel que défini ci-dessus) avec un hydrure de métal alcalin, puis avec un dérivé de formule générale (III) (dans laquelle n est tel que défini ci-dessus et Y représente un atome de brome ou de chlore).

La réaction s'effectue par exemple dans un solvant inerte tel que le diméthylformamide.

Ensuite on fait réagir le dérivé bicyclique de formule générale (IVa) ainsi obtenu avec un dérivé de pipérazine de formule générale (V) (dans laquelle X est tel que défini ci-dessus).

La réaction s'effectue par exemple dans un solvant tel que le toluène, en présence d'acide paratoluènesulfonique.

Les dérivés chlorés de formule générale (IV) dans laquelle n=2 peuvent également être obtenus par action, par exemple, du chlorure de thionyle ou du chlorure de méthanesulfonyle sur les analogues hydroxylés, eux mêmes obtenus à partir des phényl-6 1*H*,3*H*-pyrimidinediones-2,4 de formule générale (II) et de dioxolanne-1,3 one-2.

Les phényl-6 1*H*,3*H*-pyrimidinediones-2,4 de formule générale (II) sont décrites dans les brevets US-4593030, US-4625028, DE-2142317, et dans des articles de J. Org. Chem., 10, 1945-1948, (1971), Synthesis, 1, 70-72, (1988), et C.A., 106, 102199w, (1987).

La (naphtyl-1)-1 pipérazine (formule générale V, X = CH) est décrite dans J. Med. Chem., 29(11), 2379 (1986). Les (R3-7 naphtyl-1)-1 pipérazines de formule générale (V) peuvent être préparées selon la méthode décrite dans ce document, c'est-à-dire par réaction entre un amino-1 R3-7 naphtalène et la bis(chloro-2 éthyl-2)amine.

Schéma 2

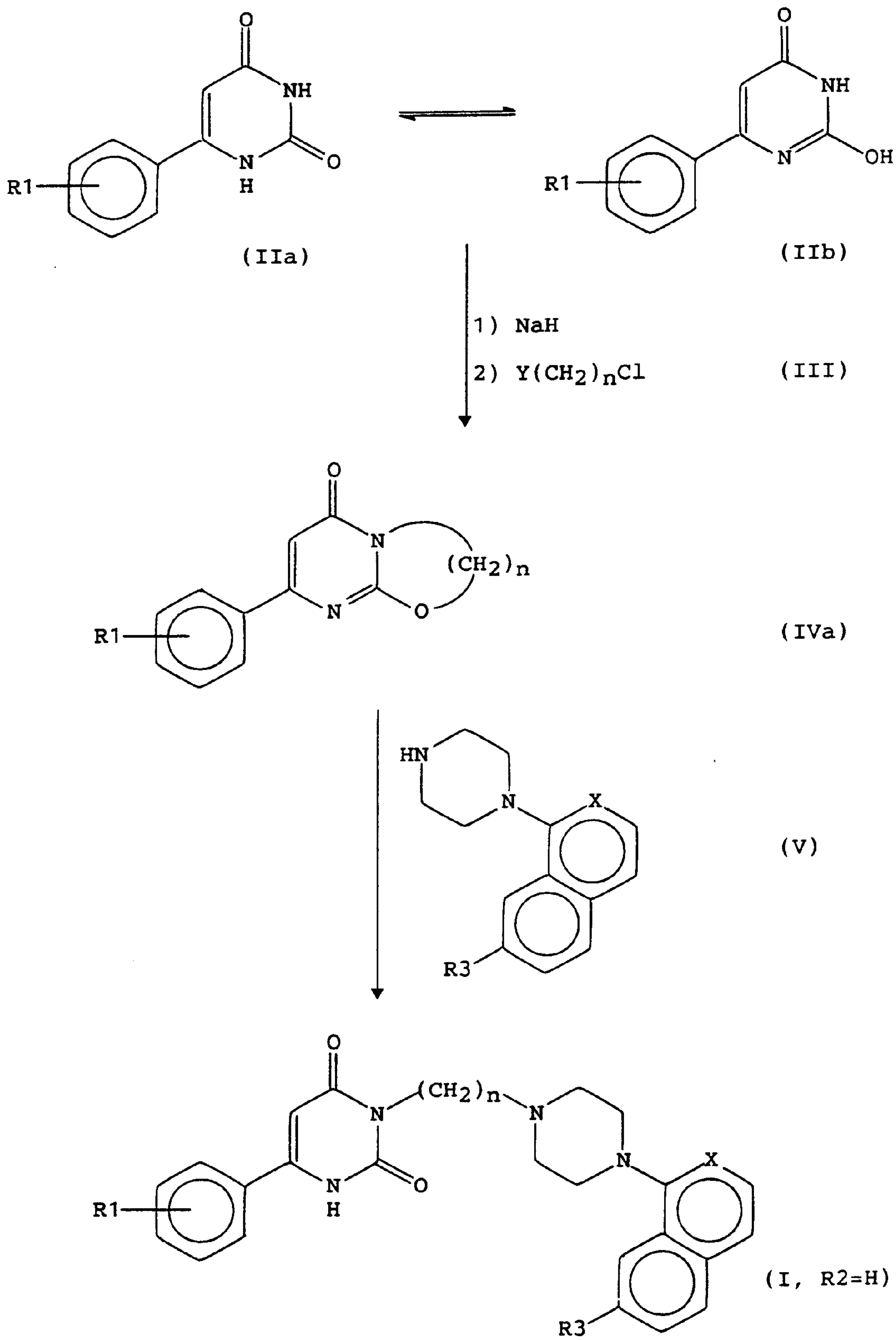


(IIa) (IIb)

(III)

(IVa)

(V)

(I, R2=H)

La (pipérazinyl-1)-1 isoquinoléine (formule générale V, X = N) est nouvelle ; elle peut être préparée en deux étapes, d'abord par action de l'oxychlorure de phosphore sur la 2*H*-isoquinoléinone-1, puis par action du produit ainsi obtenu sur la pipérazine.

Les exemples qui vont suivre illustrent en détail la préparation de quelques composés selon l'invention. Les micro-analyses et les spectres IR et RMN confirment les structures des produits obtenus.
Les numéros indiqués entre parenthèses dans les titres des exemples correspondent à ceux du tableau donné plus loin.

Le tableau donné plus loin illustre les structures chimiques et les propriétés physiques de quelques composés selon l'invention.

Exemple 1 (Composé n°4)

Méthyl-1 [[(naphtyl-1)-4 pipérazinyl-1]-2 éthyl]-3 phényl-6 1*H*,3*H*-pyrimidinedione-2,4, fumarate neutre.

a) (Hydroxy-2 éthyl)-3 méthyl-1 phényl-6 1*H*,3*H*-pyrimidinedione-2,4.

A une solution de 33,85 g (167 mmoles) de méthyl-1 phényl-6 1*H*,3*H*-pyrimidinedione-2,4 et 17,0 g (193 mmoles) de dioxolanne-1,3 one-2 dans 450 ml de diméthylformamide on ajoute 3,0 g de carbonate de potassium anhydre et on chauffe la suspension au reflux pendant 3 h.
On laisse refroidir, on sépare le produit minéral par filtration et on évapore le solvant sous vide. On recristallise le résidu dans 750 ml de toluène et, après séchage, on obtient 5,4 g de cristaux incolores.

b) (Chloro-2 éthyl)-3 méthyl-1 phényl-6 1*H*,3*H*-pyrimidinedione-2,4.

On ajoute 62,5 g, soit 86,1 ml (620 mmoles) de triéthylamine à une solution de 35,3 g (143 mmoles) de (hydroxy-2 éthyl)-3 méthyl-1 phényl-6 1*H*,3*H*-pyrimidinedione-2,4 dans 1200 ml d'acétonitrile, on refroidit la solution à 0°C et, tout en agitant sous atmosphère d'azote on ajoute une solution de 42,3 g, soit 28,7 ml (370 mmoles) de chlorure de méthane-sulfonyle dans 400 ml d'acétonitrile, de façon à maintenir la température inférieure à 5°C. On maintient l'agitation pendant 3 h à cette température, puis pendant 1 h à 10°C et pendant 1 h à 15°C.
On sépare l'insoluble par filtration, on ajoute au filtrat du dichlorométhane et une solution aqueuse de bicarbonate de sodium et on sépare, lave, sèche et évapore la phase organique. On traite le résidu par chromatographie sur colonne de silice et on obtient finalement 17,4 g de produit pur.

c) Méthyl-1 [[(naphtyl-1)-4 pipérazinyl-1)-2 éthyl]-3 phényl-6 1*H*,3*H*-pyrimidinedione-2,4.

On chauffe au reflux pendant 8 h une solution de 2,2 g (8,3 mmoles) de (chloro-2 éthyl)-3 méthyl-1 phényl-6 1*H*,3*H*-pyrimidimedione-2,4 et 3,8 g (18 mmoles) de (naphtyl-1)-1 pipérazine dans 100 ml de méthanol, puis on évapore le solvant, on ajoute de l'ammoniaque aqueuse et de l'acétate d'éthyle, on sépare la phase organique, on la lave à l'eau, on la sèche sur sulfate de sodium et on l'évapore. On purifie le résidu huileux par chromatographie sur colonne de silice, ce qui laisse 2,2 g de base libre. On dissout cette dernière dans le minimum d'éthanol, on ajoute 0,58 g d'acide fumarique, on agite le mélange puis on le laisse reposer.
On isole le sel par filtration et on le recristallise dans l'éthanol. On obtient 1,80 g de fumarate.
Point de fusion : 158 - 160°C.

Exemple 2. (Composé n°7)

[[(Naphtyl-1)-4 pipérazinyl-1]-2 éthyl]-3 phényl-6 propyl-1 1*H*,3*H*-pyrimidinedione-2,4, fumarate acide.

a) [Chloro-2 éthyl]-3 phényl-6 propyl-1 1*H*,3*H*-pyrimidinedione-2,4

On chauffe au reflux pendant 1 h un mélange de 9 g (39 mmoles) de phényl-6 propyl-1 1*H*,3*H*-pyrimidinedione-2,4, 4,36 g de potasse, 0,43 g de chlorure de triéthylbenzylammonium et 200 ml de dichloro-1,2 éthane. On sépare l'insoluble par filtration, on évapore la phase liquide et on purifie le résidu par chromatographie sur colonne de silice en éluant avec un mélange 99/1 de dichlorométhane/méthanol. On obtient 10 g de produit huileux qu'on utilise tel quel dans l'étape suivante.

b) [[(Naphtyl-1)-4 pipérazinyl-1]-2 éthyl]-3 phényl-6 propyl-1 1*H*,3*H*-pyrimidinedione-2,4.

On chauffe au bain d'huile, à 120°C, pendant 3 h, un mélange de 2,92 g (10 mmoles) de [chloro-2 éthyl]-3 phényl-6 propyl-1 1*H*,3*H*-pyrimidinedione-2,4 et 4,67 g (22 mmoles) de (naphtyl-1)-1 pipérazine. On laisse refroidir, on reprend le produit cristallisé avec un mélange d'acétate d'éthyle et d'ammoniaque 3N, on sépare la phase organique, on la lave à l'eau, on la sèche sur sulfate de sodium et on évapore le solvant. On purifie l'huile résiduelle par chromatographie sur colonne de silice en éluant avec un mélange 98/2 de dichlorométhane/méthanol.
On obtient ainsi 3,56 g (7,6 mmoles) de base qu'on dissout dans l'acétone, on ajoute 0,88 g (7,6 mmoles) d'acide fumarique, on agite le mélange pendant 1 h, on essore le précipité et on le recristallise dans l'acétone. On isole finalement 2,3 g de fumarate.
Point de fusion : 182-184°C.

Exemple 3. (Composé n°12)

Méthyl-1 (méthyl-3 phényl)-6 [[(naphtyl-1)-4 pipérazinyl-1]-2 éthyl]-3 1*H*,3*H*-pyrimidinedione-2,4, fumarate acide.

a) Méthyl-1 (méthyl-3 phényl)-6 1*H*,3*H*-pyrimidinedione-2,4.

On ajoute goutte à goutte une solution de 35 g (237 mmoles) de *N*-méthyl (méthyl-3 phényl)-1 éthanimine dans 150 ml de chlorobenzène à 25 g, soit 9,88 ml (237 mmoles) d'isocyanate de chlorocarbonyle, puis on chauffe le mélange au reflux pendant 4 h. On évapore le solvant, on reprend le résidu avec de l'hexane, on agite le mélange pendant 3 h, on le filtre et on purifie le solide par chromatographie sur colonne de silice en éluant avec un mélange 98/2 de dichlorométhane/méthanol. On obtient 11,9 g de produit qu'on utilise tel quel dans l'étape suivante.

b) (Chloro-2 éthyl)-3 méthyl-1 (méthyl-3 phényl)-6 1*H*,3*H*-pyrimidinedione-2,4.

On chauffe au reflux pendant 2 h un mélange de 11,9 g (55 mmoles) de méthyl-1 (méthyl-3 phényl)-6 1*H*,3*H*-pyrimidinedione-2,4, 200 ml de dichloro-1,2 éthane, 0,6 g de chlorure de triéthylbenzylammonium et 6,16 g de potasse pulvérisée. On filtre le mélange, on évapore le filtrat et on purifie l'huile résiduelle par chromatographie sur colonne de silice en éluant avec un mélange 98/2 de dichlorométhane/méthanol. On obtient 11 g de composé cristallisé.

c) Méthyl-1 (méthyl-3 phényl)-6 [[(naphtyl-1)-4 pipérazinyl-1]-2 éthyl]-3 1*H*,3*H*-pyrimidinedione-2,4.

On mélange au mortier 4,18 g (15 mmoles) de (chloro-2 éthyl)-3 méthyl-1 (méthyl-3 phényl)-6 1*H*,3*H*-pyrimidinedione-2,4 et 6,79 g (32 mmoles) de (naphtyl-1)-1 pipérazine. On place ce mélange dans un ballon qu'on chauffe au bain d'huile à 110°C pendant 3 h.
On le laisse refroidir, on reprend le produit cristallisé avec un mélange d'acétate d'éthyle et d'ammoniaque 3N, on sépare la phase organique, on la lave à l'eau, on la sèche sur sulfate de sodium et on évapore le solvant. On purifie l'huile résiduelle par chromatographie sur colonne de silice en éluant avec un mélange 98/2 de dichlorométhane/méthanol. On obtient ainsi 5,75 g de base qu'on dissout dans de l'éthanol chaud, on ajoute une solution de 1,47 g d'acide fumarique dans le minimum d'éthanol, on filtre le sel qui précipite et on le recristallise dans l'éthanol. On isole finalement 5 g de fumarate.
Point de fusion : 178°C.

Exemple 4. (Composé n°15)

Méthyl-1 [[(naphtyl-1)-4 pipérazinyl-1]-3 propyl]-3 phényl-6 1*H*,3*H*-pyrimidinedione-2,4, chlorhydrate.

a) (Chloro-3 propyl)-3 méthyl-1 phényl-6 1*H*,3*H*-pyrimidinedione-2,4.

On chauffe au reflux pendant 8 h un mélange de 17,5 g (86 mmoles) de méthyl-1 phényl-6 1*H*,3*H*-pyrimidinedione-2,4, 5,7 g de potasse et 300 ml de bromo-1 chloro-3 propane. On évapore le solvant, on reprend le résidu avec de l'eau, on l'extrait à l'acétate d'éthyle, on lave la phase organique à l'eau, on la sèche sur sulfate de sodium et on l'évapore.

On obtient 13 g de résidu huileux qu'on purifie par chromatographie sur colonne de silice en éluant avec un mélange 98/2 de dichlorométhane/méthanol. On isole ainsi 2,85 g de produit solide constitué de 94% de (chloro-3 propyl)-3 méthyl-1 phényl-6 1*H*,3*H*-pyrimidinedione-2,4, le reste étant l'analogue bromé.

b) Méthyl-1 [[(naphtyl-1)-4 pipérazinyl-1]-3 propyl]-3 phényl-6 1*H*,3*H*-pyrimidinedione-2,4.

On mélange au mortier 2,85 g (10,2 mmoles) de (chloro-3 propyl)-3 méthyl-1 phényl-6 1*H*,3*H*-pyrimidinedione-2,4 et 5,19 g (24,5 mmoles) de (naphtyl-1)-1 pipérazine.
On place ce mélange dans un ballon qu'on chauffe au bain d'huile à 100°C pendant 1 h.
On broie le produit obtenu et on le reprend avec un mélange d'acétate d'éthyle et d'ammoniaque 3N, on sépare la phase organique, on la lave à l'eau, on la sèche sur sulfate de sodium et on évapore le solvant. On purifie l'huile résiduelle par chromatographie sur colonne de silice en éluant avec un mélange 98/2 de dichlorométhane/méthanol.
On obtient ainsi 3,4 g de base qu'on dissout dans le minimum d'acétone et on ajoute de l'éther chlorhydrique jusqu'à pH acide. Le chlorhydrate précipite, on l'agite encore pendant 1 h, on le filtre et on le recristallise dans un mélange d'acétone et d'éthanol. On isole finalement 1,9 g de chlorhydrate.
Point de fusion : 268-270°C (avec décomposition).

Exemple 5. (Composé n°17)

[[(Méthoxy-7 naphtyl-1)-4 pipérazinyl-1]-2 éthyl]-3 méthyl-1 phényl-6 1*H*,3*H*-pyrimidinedione-2,4 fumarate acide.

On chauffe au bain d'huile, pendant 2 h, un mélange de 1,32 g (5 mmoles) de (chloro-2 éthyl)-3 méthyl-1 phényl-6 1*H*,3*H*-pyrimidinedione-2,4 et 2,42 g (10 mmoles) de (méthoxy-7 naphtyl-1)-1 pipérazine. on laisse refroidir, on reprend le produit cristallisé avec un mélange d'acétate d'éthyle et d'ammoniaque 3N, on sépare la phase organique, on la lave à l'eau, on la sèche sur sulfate de sodium et on évapore le solvant. On purifie l'huile résiduelle par chromatographie sur colonne de silice en éluant avec un mélange 98/2 de dichlorométhane/méthanol.
On obtient ainsi 1,9 g (4 mmoles) de base qu'on dissout dans l'éthanol, on ajoute 0,46 g (4 mmoles) d'acide fumarique, on agite le mélange, on essore le précipité et on le recristallise dans l'éthanol. On isole finalement 1,64 g de fumarate.
Point de fusion : 222-224°C.

Exemple 6. (Composé n°2)

[[(Fluoro-7 naphtyl-1)-4 pipérazinyl-1]-2 éthyl]-3 phényl-6 1*H*,3*H*-pyrimidinedione-2,4.

a) (Fluoro-7 naphtyl-1)-1 pipérazine.

Dans un ballon de 500 ml muni d'un appareil de Dean-Stark et placé sous atmosphère inerte on introduit 30,0 g (186 mmoles) de fluoro-7 naphtalèneamine-1 et 33,22 g (186 mmoles) de chlorhydrate de bis(chloro-2 éthyl-2)amine en solution dans 170 ml de butanol-1. On ajoute un peu d'iodure de potassium, puis on chauffe le mélange au reflux pendant 20h.
On ajoute 11,8 g de carbonate de potassium, on chauffe au reflux pendant 10h, on ajoute 3,87 g de carbonate de potassium et on chauffe encore au reflux pendant 10h, et on renouvelle cette dernière opération encore deux fois.
On évapore le solvant sous pression réduite, on reprend le résidu avec de l'eau et de l'éther, on agite le mélange, on essore la suspension obtenue, et on recristallise le solide dans un mélange 80/20 d'eau/éthanol. On en libère la base en l'agitant dans de l'eau et en ajoutant de l'hydroxyde de sodium, et on l'extrait avec de l'éther. Après séchage et évaporation de la phase organique on obtient une huile qui cristallise. Point de fusion : 46,5-47,5°C.

b) Phényl-7 dihydro-2,3 5*H*-oxazolo[3,2-*a*]pyrimidinone-5.

A une suspension de 1,45 g (30 mmoles) d'hydrure de sodium à 50% dans l'huile (préalablement lavé avec trois fois 20 ml de pentane sec) dans 60 ml de diméthylformamide sec on ajoute, par petites portions, 5,65 g (30 mmoles) de phényl-6 1*H*,3*H* pyrimidinedione-2,4, et on agite le mélange à température ambiante pendant

1h, jusqu'à cessation du dégagement gazeux. On refroidit le mélange avec un bain de glace, et on ajoute, en une seule fois, 7,5 ml (90 mmoles) de bromo-1 chloro-2 éthane. On chauffe le mélange à environ 70°C pendant 15h, on évapore le solvant, on reprend le résidu avec de l'eau et du dichlorométhane, ce qui laisse apparaître un solide cristallin qu'on essore, lave et sèche. On récupère ainsi 2,5 g de phényl-6 1*H*,3*H*-pyrimidinedione-2,4 qui n'a pas réagi. Par ailleurs on sépare la phase organique du filtrat, on la lave et on la sèche sur sulfate de magnésium, et on l'évapore. On obtient 4 g d'une huile qu'on purifie par chromatographie sur colonne de silice avec un mélange 96/4 de dichlorométhane/ méthanol. Après recristallisation dans l'éther on isole 1,66 g de produit pur. Point de fusion : 162-163°C.

c) [[(Fluoro-7 naphtyl-1)-4 pipérazinyl-1]-2 éthyl]-3 phényl-6 1*H*,3*H*-pyrimidinedione-2,4.

On mélange 1,28 g (6 mmoles) de phényl-7 dihydro-2,3 5*H*-oxazolo[3,2-a]pyrimidinone-5 avec 1,38 g de (fluoro-7 naphtyl-1)-1 pipérazine dans 25 ml de toluène en présence de quelques milligrammes d'acide paratoluènesulfonique, puis on chauffe le mélange au reflux pendant 20h.
On essore le solide qui s'est formé, on le lave avec de l'éther, et on le reprend avec de l'eau et du dichlorométhane. On lave la phase organique, on la sèche sur sulfate de magnésium, on l'évapore, et on agite le résidu dans de l'éthanol bouillant avant de l'essorer et de le sécher. On isole finalement 1,65 g de produit pur.
Point de fusion : 223-224°C.

Exemple 7. (Composé n°25)

[[(Isoquinoléinyl-1)-4 pipérazinyl-1]-2 éthyl]-3 méthyl-1 (méthyl-2 phényl)-6 1*H*,3*H*-pyrimidinedione-2,4, sesquifumarate.

a) (Pipérazinyl-1)-1 isoquinoléine.

A une suspension de 52,8 g (363 mmoles) de 2*H*-isoquinoléinone-1 dans 250 ml d'acétonitrile on ajoute goutte à goutte 65 ml d'oxychlorure de phosphore, on chauffe le mélange au reflux pendant 3 h, et on évapore le solvant. On dissout le résidu (305 mmoles) dans du méthanol, on ajoute une solution de 75 g (870 mmoles) de pipérazine dans du méthanol, et on évapore le solvant. On chauffe le résidu à 120°C pendant 4 h, on laisse refroidir, on ajoute 500 ml d'eau, on agite le mélange, on le filtre, on extrait le filtrat à l'acétate d'éthyle. Après séparation, lavage à l'eau, séchage et évaporation de la phase organique on obtient 57 g de base sous forme d'huile.
On la dissout dans 400 ml d'éthanol, on ajoute 30,7 g d'acide fumarique, on chauffe le mélange au reflux pendant 30 mn, on le laisse reposer, on sépare le précipité par filtration et on le recristallise dans un mélange 1/1 d'éthanol/eau. On isole finalement 64 g de fumarate.
Point de fusion : 201-203°C.

b) (Chloro-2 éthyl)-3 méthyl-1 (méthyl-2 phényl)-6 1*H*,3*H*-pyrimidinedione-2,4.

On chauffe au reflux pendant 2 h 30 un mélange de 10,8 g (50 mmoles) de méthyl-1 (méthyl-2 phényl)-6 1*H*,3*H*-pyrimidinedione-2,4, 0,5 g de chlorure de triéthylbenzylammonium, 5,6 g de potasse pulvérisée et 300 ml de dichloro-1,2 éthane.
On filtre le mélange, on évapore le filtrat, et on purifie l'huile résiduelle par chromatographie sur colonne de silice en éluant avec un mélange 98/2 de dichlorométhane/méthanol. On obtient 10,9 g de produit huileux qu'on utilise tel quel dans l'étape suivante.

c) [[(Isoquinoléinyl-1)-4 pipérazinyl-1]-2 éthyl]-3 méthyl-1 (méthyl-2 phényl)-6 1*H*,3*H*-pyrimidinedione-2,4.

On chauffe au bain d'huile à 120°C pendant 2 h un mélange de 3,6 g (13 mmoles) de (chloro-2 éthyl)-3 méthyl-1 (méthyl-2 phényl)-6 1*H*,3*H*-pyrimidinedione-2,4 et la base libre obtenue à partir de 8,5 g (26 mmoles) de fumarate de (pipérazinyl-1)-1 isoquinoléine.
On laisse refroidir le mélange, on le reprend avec de l'ammoniaque 3N et on l'extrait avec trois fois 100 ml d'acétate d'éthyle. On lave la phase organique, on la sèche sur sulfate de sodium et on l'évapore. On purifie l'huile résiduelle par chromatographie sur colonne de silice en éluant avec un mélange 95/5 de dichlorométhane/méthanol. On obtient ainsi 5,06 g (11 mmoles) de base pure qu'on dissout dans l'éthanol, on ajoute 2,76 g (22 mmoles) d'acide fumarique en solution dans l'éthanol, et on concentre la solution. On filtre le précipité qui se forme et on le recristallise dans l'éthanol. On isole finalement 5,49 g de sesquifumarate solvaté avec 1

mole d'éthanol pour 1 mole de sel.
Point de fusion : 135-137°C.

Tableau

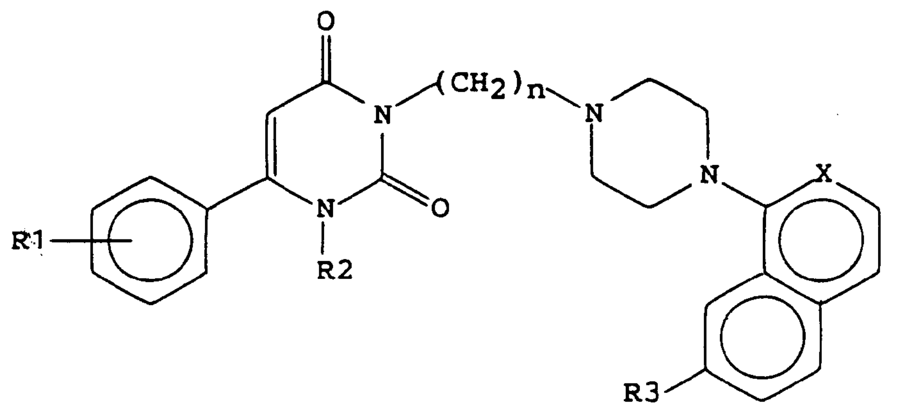

(I)

| N° | R1 | R2 | n | X | R3 | Sel/base | F(°C) |
|---|---|---|---|---|---|---|---|
| 1 | H | H | 2 | CH | H | base | 221-222 |
| 2 | H | H | 2 | CH | F | base | 223-224 |
| 3 | H | H | 2 | CH | $OCH_3$ | base | 250-252 |
| 4 | H | $CH_3$ | 2 | CH | H | fum. | 158-160 |
| 5 | H | $CH_3$ | 2 | CH | F | base | 146-147 |
| 6 | H | $C_2H_5$ | 2 | CH | H | fum. ac. | 180-181 |
| 7 | H | $nC_3H_7$ | 2 | CH | H | fum. ac. | 182-184 |
| 8 | H | $CH_2C_6H_5$ | 2 | CH | H | fum. | 162 |
| 9 | 4-F | $CH_3$ | 2 | CH | H | base | 156-157 |
| 10 | 4-Cl | $CH_3$ | 2 | CH | H | base | 156-158 |
| 11 | 2-$CH_3$ | $CH_3$ | 2 | CH | H | fum. | 196-196,5 |
| 12 | 3-$CH_3$ | $CH_3$ | 2 | CH | H | fum. ac. | 178 |
| 13 | 4-$CH_3$ | $CH_3$ | 2 | CH | H | fum. | 182-184 |
| 14 | 4-$OCH_3$ | $CH_3$ | 2 | CH | H | base | 132-134 |
| 15 | H | $CH_3$ | 3 | CH | H | HCl | 268-270 |
| 16 | H | $CH_3$ | 4 | CH | H | fum. ac. | 160 |
| 17 | H | $CH_3$ | 2 | CH | $OCH_3$ | fum. ac. | 222-224 |
| 18 | 2-Cl | $CH_3$ | 2 | CH | H | fum. | 184-186 |
| 19 | 2-$CH_3$ | $CH_3$ | 4 | CH | H | fum.ac. | 178-180 |
| 20 | 2-F | $CH_3$ | 2 | CH | H | fum. | 160-162 |

Tableau (suite)

| N° | R1 | R2 | n | X | R3 | Sel/base | F(°C) |
|---|---|---|---|---|---|---|---|
| 21 | H | H | 2 | N | H | base | 219-220 |
| 22 | H | $CH_3$ | 2 | N | H | base | 138-139 |
| 23 | 4-F | $CH_3$ | 2 | N | H | base | 136-137 |
| 24 | 4-Cl | $CH_3$ | 2 | N | H | base | 172-174 |
| 25 | 2-$CH_3$ | $CH_3$ | 2 | N | H | 1½fum.* | 135-137 |
| 26 | 4-$CH_3$ | $CH_3$ | 2 | N | H | fum. | 180-182 |
| 27 | 4-$OCH_3$ | $CH_3$ | 2 | N | H | base | 160-161 |
| 28 | H | $CH_3$ | 4 | N | H | fum. ac. | 158 |

<u>Légende du tableau</u>

HCl : chlorhydrate (base/acide = 1/1)

fum. : fumarate neutre (base/diacide = 2/1)

fum. ac. : fumarate acide (base/diacide = 1/1)

1½fum. : sesquifumarate (base/diacide = 2/3)

* : solvaté (sel/éthanol = 1/1)

Les composés de l'invention ont été soumis a une série d'essais pharmacologiques qui ont mis en évidence leur intérêt comme substances à activités thérapeutiques.

Ainsi ils ont fait l'objet d'une étude quant à leur affinité pour les récepteurs sérotoninergiques du type 5-$HT_{1A}$ présents dans l'hippocampe du rat.

Les composés déplacent la liaison d'un ligand spécifique marqué, la [$^3$H]-hydroxy-8 di-n-propylamino-2 tétraline (désignée ci-après par "[$^3$H]-8-OH-DPAT" et décrite par Gozlan et coll., Nature, (1983), <u>305</u>, 140-142) sur les récepteurs 5-$HT_{1A}$.

Les animaux utilisés sont des rats mâles Sprague-Dawley de 160 à 200 g. Après décapitation on en prélève le cerveau et on excise l'hippocampe. On broie le tissu dans un appareil Ultra-Turrax Polytron™ pendant 30 s à la moitié de la vitesse maximale dans 10 volumes de tampon Tris 50 mM d'un pH ajusté à 7,4 avec de l'acide chlorhydrique (soit 100 mg de tissu frais par ml). On lave les tissus homogénéisés trois fois à 4°C, en les centrifugeant à chaque fois pendant 10 mn à 48000xg et en remettant le culot en suspension dans du tampon frais refroidi. Finalement on met le dernier culot en suspension dans le tampon pour arriver à une concentration de 100 mg de tissu de départ par ml de tampon à 50 mM.

On laisse ensuite incuber à 37°C pendant 10 mn.

La liaison avec la [$^3$H]-8-OH-DPAT (1 nM) est déterminée par incubation de 100 μl de suspension de membranes dans un volume final de 1 ml de tampon contenant 10 μM de pargyline et 3 μM de paroxétine.

Après une incubation de 15 mn à 37°C on récupère les membranes par filtration sur filtres Whatman GF/B™ qu'on lave trois fois avec des quantités aliquotes de 5 ml de tampon glacé. On extrait les filtres dans le liquide de scintillation et on en mesure la radioactivité par scintigraphie liquide. La liaison spécifique de la [$^3$H]-8-OH-DPAT est définie comme la quantité radioactive retenue sur les filtres et pouvant être inhibée par co-incubation dans la hydroxy-5 tryptamine à 10 μM. A une concentration de 1 nM de [$^3$H]-8-OH-DPAT la liaison spécifique représente 908 de la radioactivité totale recupérée sur le filtre.

Pour chaque concentration de composés étudié on détermine le pourcentage d'inhibition de la liaison avec la [$^3$H]-8-OH-DPAT, puis la concentration $CI_{50}$, concentration qui inhibe 50% de la liaison.

Pour les composés de l'invention les $CI_{50}$ se situent entre 0,001 et 0,1 µM.

Les composés de l'invention ont aussi fait l'objet d'un essai de déplacement de la liaison (binding) du spiropéridol sur les récepteurs sérotoninergiques (5-$HT_2$) du cortex cérébral du rat.

Pour cet essai on prélève les cerveaux de rats, on en dissèque le cortex et on l'homogénéise à 0°C dans 10 volumes d'un mélange contenant, par litre, 50 millimoles de tampon Tris/HCl à pH = 7,4, 120 millimoles de chlorure de sodium et 5 millimoles de chlorure de potassium. On centrifuge le mélange homogène à 40000xg pendant 10 mn puis, à deux reprises, on récupère le culot, on le lave en le mettant en suspension dans le même mélange tampon, on l'homogénéise de nouveau et on le centrifuge. Pour terminer on dilue le culot final dans le même mélange tampon à raison de 100 mg de tissu humide pour 1 ml de tampon.

On soumet alors le tissu à une incubation préalable de 10 mn à 37°C en présence de 10 micromoles/l de pargyline, puis à une incubation de 20 mn à 37°C en présence de $^3$H-spiropéridol (activité spécifique : 25,6 Ci par millimole) à la concentration de 0,3 nanomole/l et de composé à étudier à des concentrations allant de 0,0001 à 100 micromoles/l.

On prélève des aliquots de 1 ml que l'on filtre sous vide, on lave les filtres deux fois avec 5 ml de tampon froid et on les sèche. La radioactivité est mesurée dans le toluène en présence de 5 g/l de diphényl-2,5 oxazole (PPO) et 0,1 g/l de bis-(phényl-5 oxazolyl-2)-1,4 benzène (POPOP).

Pour évaluer l'activité des composés on établit la courbe du pourcentage d'inhibition de la liaison spécifique de $^3$H-spiropéridol en fonction de la concentration en drogue déplaçante. On détermine graphiquement la concentration $CI_{50}$, concentration qui inhibe 50 % de la liaison spécifique.

La liaison spécifique est définie comme étant la liaison déplacée par 100 micromoles/1 de 5-HT.

Les concentrations $CI_{50}$ des composés de l'invention se situent pour la plupart entre 0,1 et 0,5 µM.

L'activité centrale des composés de l'invention a été évaluée par leurs effets sur les "pointes PGO" (ponto-géniculo-occipitales) induites par la réserpine (test PGO-R) chez le chat, selon la méthode décrite par H. Depoortere, Sleep 1976, 3rd Europ. Congr. Sleep Res., Montpellier 1976, 358-361 (Karger, Basel 1977).

On administre des doses cumulatives de composés à étudier (de 0,001 à 3 mg/kg par voie intraveineuse) à des intervalles de temps de 30 mn, 4h après injection intrapéritonéale d'une dose de 0,75 mg/kg de réserpine, à des chats curarisés, sous ventilation artificielle. On recueille les activités électro-encéphalographique et phasique (pointes PGO-R) à l'aide d'électrodes corticales et profondes (genouillé latéral).

Pour chaque dose de composé étudié on détermine le pourcentage de diminution du nombre de pointes PGO, puis la $DE_{50}$, dose active qui diminue de 50% ce nombre de pointes.

Pour les composés de l'invention les $DE_{50}$ se situent entre 0,09 et 3 mg/kg par la voie intraveineuse.

Enfin les composés de l'invention ont été soumis au test de l'ischémie cérébrale globale chez la souris. L'ischémie est due à un arrêt cardiaque induit par une injection intraveineuse rapide de chlorure de magnésium. Dans ce test on mesure le "temps de survie", c'est-à-dire l'intervalle entre le moment de l'injection de chlorure de magnésium et le dernier mouvement respiratoire observable de chaque souris. Ce dernier mouvement est considéré comme l'indice ultime d'une fonction du système nerveux central.

L'arrêt respiratoire apparaît approximativement 19 secondes après l'injection de chlorure de magnésium.

Des souris mâles (Charles River CD1) sont étudiées par groupes de 10. Elles sont nourries et abreuvées ad libitum avant les essais. Le temps de survie est mesuré 10 minutes après l'administration intrapéritonéale des composés de l'invention. Les résultats sont donnés sous la forme de la différence entre le temps de survie mesuré dans un groupe de 10 souris ayant reçu le composé et le temps de survie mesuré dans un groupe de 10 souris ayant reçu le liquide véhicule. Les rapports entre les modifications dans le terme de survie et la dose du composé sont enregistrés graphiquement selon une courbe semilogarithmique.

Cette courbe permet le calcul de la dose effective 3 secondes ($DE_{3"}$), c'est-à-dire la dose (en mg/kg) qui produit une augmentation de 3 secondes du temps de survie par rapport au groupe témoin de 10 souris non traitées. Une augmentation de 3 secondes du temps de survie est à la fois significative statistiquement et reproductible. Les $DE_{3"}$ des meilleurs composés de l'invention (dans ce test) sont de l'ordre de 5 mg/kg par voie intrapéritonéale.

Les résultats des essais montrent que les composés de formule générale (I) possèdent, in vitro, une grande affinité et une sélectivité pour les récepteurs sérotoninergiques de type 5-$HT_{1A}$. In vivo ils montrent une activité agoniste ou agoniste partielle, au niveau de ces récepteurs.

Les composés de l'invention peuvent donc être utilisés pour le traitement des maladies et affections impliquant les récepteurs sérotoninergiques de type 5-$HT_{1A}$ de façon directe ou indirecte, notamment pour le traitement des états psychotiques (schizophrénies), des états dépressifs, des états d'anxiété, des troubles du sommeil, des troubles du comportement sexuel, et pour la régulation de la prise de nourriture, ainsi que pour le traitement de désordres vasculaires ou cardiovasculaires tels que la migraine et l'hypertension.

A cet effet ils peuvent être présentés sous toutes formes appropriées à leur administration par voie orale ou parentérale, associés à tous excipients convenables, et dosés pour permettre une posologie journalière de

1 à 1000 mg.

## Revendications

### Revendications pour les Etats contractants suivants : AT, BE, CH, DE, FR, GB, IT, LI, LU, NL, SE

1. Composés répondant à la formule générale (I)

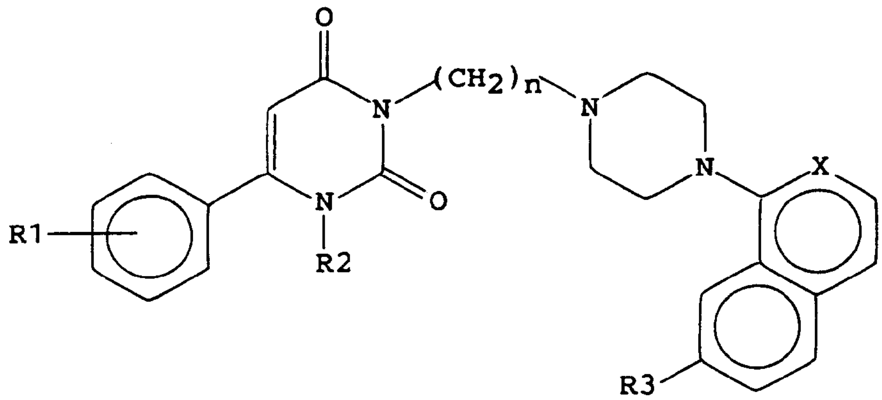

(I)

dans laquelle
R1 représente un atome d'hydrogène ou d'halogène ou un groupe méthyle ou méthoxy,
R2 représente un atome d'hydrogéne, un groupe alkyle en $C_1$-$C_4$, ou un groupe benzyle,
n représente un nombre entier égal à 2, 3 ou 4, et
X représente un groupe CH ou un atome d'azote, et
R3 représente un atome d'hydrogène ou d'halogène ou un groupe méthoxy lorsque X représente un groupe CH, ou seulement un atome d'hydrogène lorsque X représente un atome d'azote,
ainsi que leurs sels d'addition à des acides acceptables en pharmacologie.

2. Procédé de préparation des composés selon la revendication 1, caractérisé en ce qu'on fait réagir une phényl-6 1*H*,3*H*pyrimidinedione-2,4 de formule générale (II)

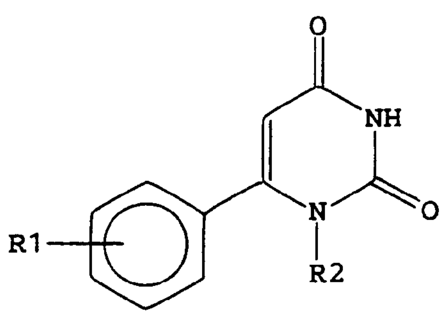

(II)

(dans laquelle R1 et R2 sont tels que définis dans la revendication 1)
avec un dérivé dihalogéné de formule générale (III)

$$Y(CH_2)_nCl \qquad (III)$$

(dans laquelle n est tel que défini dans la revendication 1 et Y représente un atome de brome ou de chlore), puis on fait réagir le dérivé de formule générale (IV) ainsi obtenu

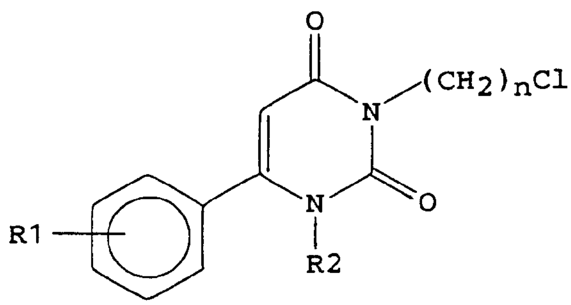

(IV)

avec un dérivé de pipérazine de formule générale (V)

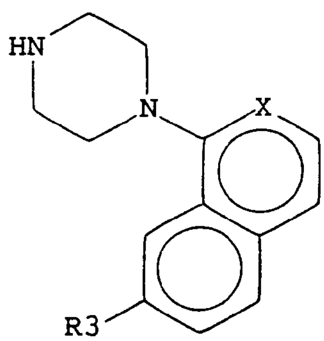

(V)

(dans laquelle X et R3 sont tels que définis dans la revendication 1).

3. Procédé de préparation des composés selon la revendication 1, dans la formule générale (I) desquels R2 représente un atome d'hydrogène, procédé caractérisé en ce qu'on fait réagir une phényl-6 1*H*,3*H*-pyrimidinedione-2,4 de formule générale (IIa, IIb)

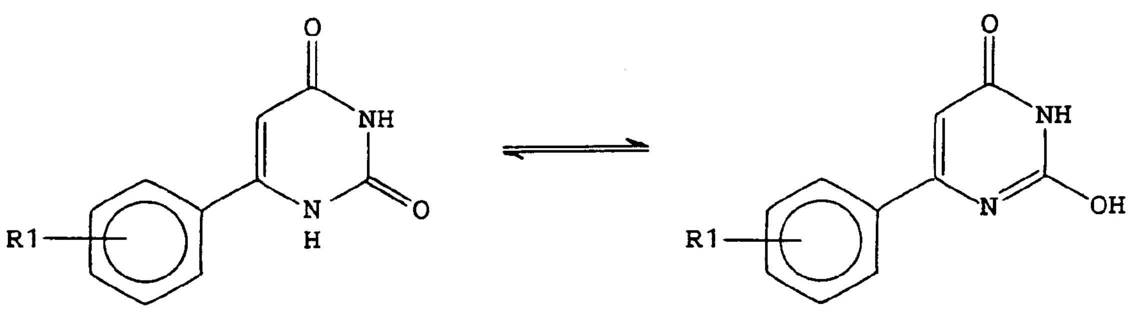

(IIa) (IIb)

(dans laquelle R1 est tel que défini dans la revendication 1) d'abord avec un hydrure de métal alcalin puis avec un dérivé dihalogéné de formule générale (III) (dans laquelle n est tel que défini dans la revendication 1 et Y représente un atome de brome ou de chlore),
puis on fait réagir le dérivé bicyclique de formule générale (IVa) ainsi obtenu

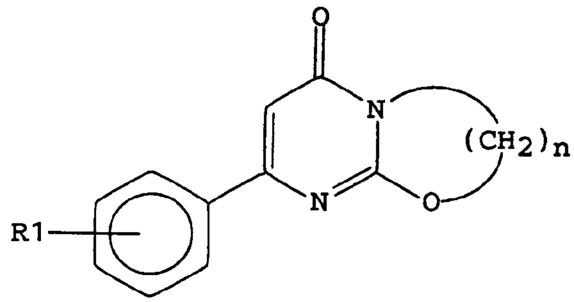

(IVa)

avec un dérivé de pipérazine de formule générale (V)

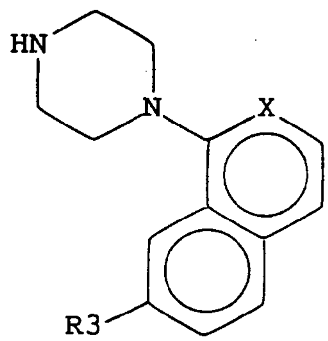

(V)

(dans laquelle X et R3 sont tels que définis dans la revendication 1).

4. Composition pharmaceutique caractérisée en ce qu'elle contient un composé selon la revendication 1, en association avec un excipient approprié.

5. La (pipérazinyl-1)-1 isoquinoléine, en tant que composé nécessaire comme intermédiaire dans le procédé selon la revendication 2.

6. La (fluoro-7 naphtyl-1)-1 pipérazine, en tant que composé nécessaire comme intermédiaire dans le procédé selon la revendication 2.

**Revendications pour les Etats contractants suivants: ES, GR**

1. Procédé de préparation de composés répondant à la formule générale (I)

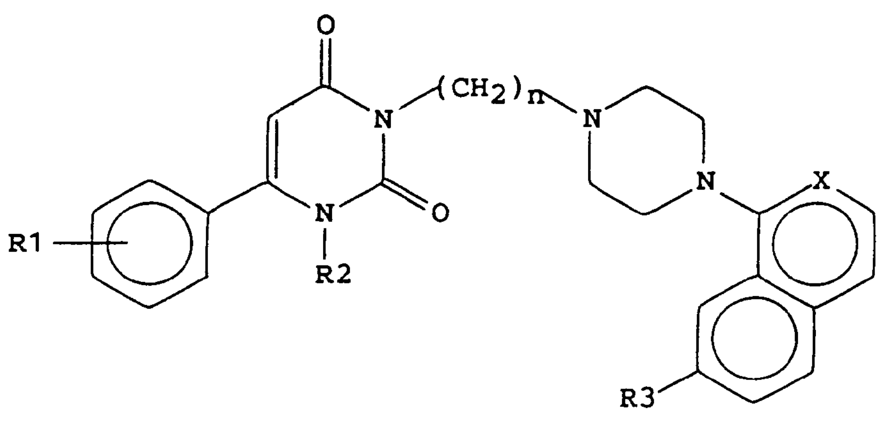

(I)

dans laquelle
R1 représente un atome d'hydrogène ou d'halogène ou un groupe méthyle ou méthoxy,
R2 représente un atome d'hydrogène, un groupe alkyle en $C_1$-$C_4$, ou un groupe benzyle,

n représente un nombre entier égal à 2, 3 ou 4, et
X représente un groupe CH ou un atome d'azote, et
R3 représente un atome d'hydrogène ou d'halogène ou un groupe méthoxy lorsque X représente un groupe CH, ou seulement un atome d'hydrogène lorsque X représente un atome d'azote,
procédé caractérisé en ce qu'on fait réagir une phényl-6 1*H*,3*H*pyrimidinedione-2,4 de formule générale (II)de formule générale (II)

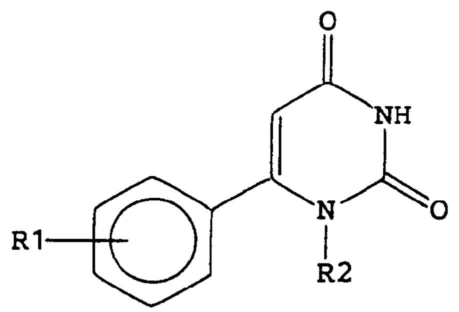

(II)

(dans laquelle R1 et R2 sont tels que définis ci-dessus) avec un dérivé dihalogéné de formule générale (III)

$Y(CH_2)_nCl$ (III)

(dans laquelle n est tel que défini ci-dessus et Y représente un atome de brome ou de chlore),
puis on fait réagir le dérivé de formule générale (IV) ainsi obtenu

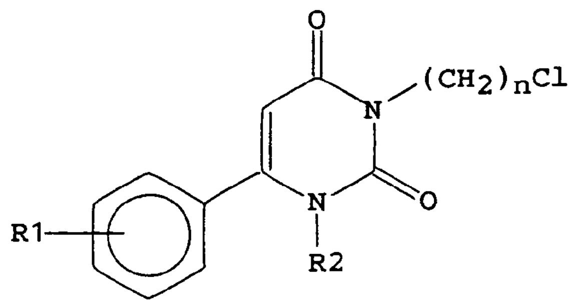

(IV)

avec un dérivé de pipérazine de formule générale (V)

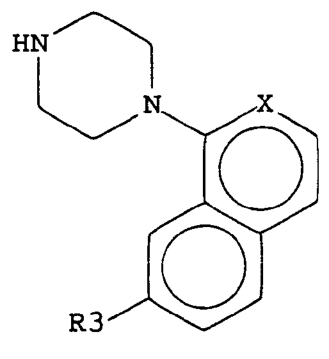

(V)

(dans laquelle X et R3 sont tels que définis ci-dessus).

**2.** Procédé de préparation des composés définis dans la revendication 1, et dans la formule générale (I) desquels R2 représente un atome d'hydrogène, procédé caractérisé en ce qu'on fait réagir une phényl-6 1*H*,3*H*-pyrimidinedione-2,4 de formule générale (IIa, IIb)

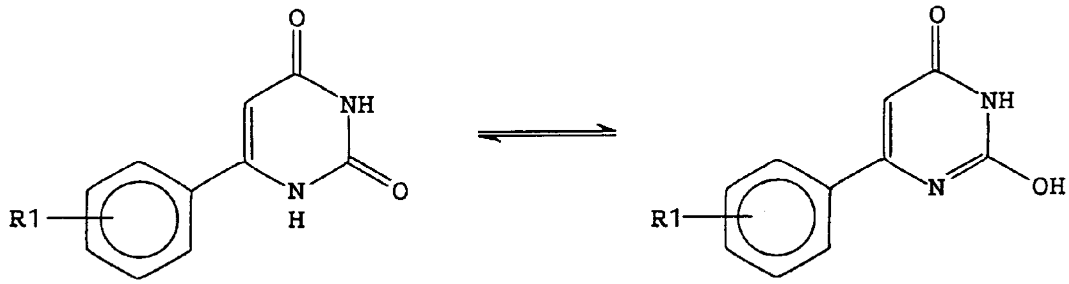

(IIa) (IIb)

(dans laquelle R1 est tel que défini dans la revendication 1) d'abord avec un hydrure de métal alcalin puis avec un dérivé dihalogéné de formule générale (III) (dans laquelle n est tel que défini dans la revendication 1 et Y représente un atome de brome ou de chlore),
puis on fait réagir le dérivé bicyclique de formule générale (IVa) ainsi obtenu

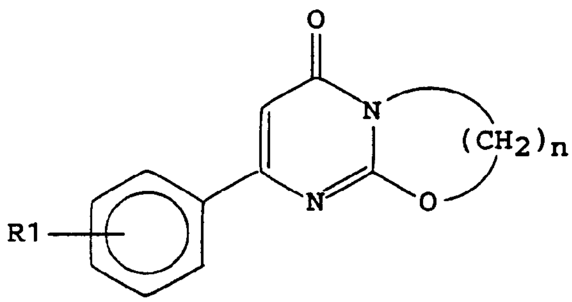

(IVa)

avec un dérivé de pipérazine de formule générale (V)

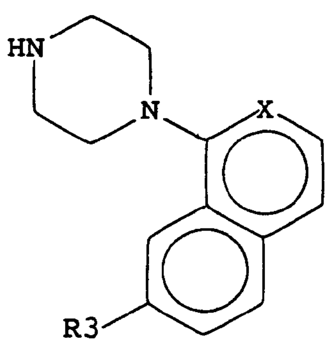

(V)

(dans laquelle X et R3 sont tels que définis dans la revendication 1).

## Patentansprüche

### Patentansprüche für folgende Vertragsstaaten : AT, BE, CH, DE, FR, GB, IT, LI, LU, NL, SE

**1.** Verbindungen der allgemeinen Formel (I)

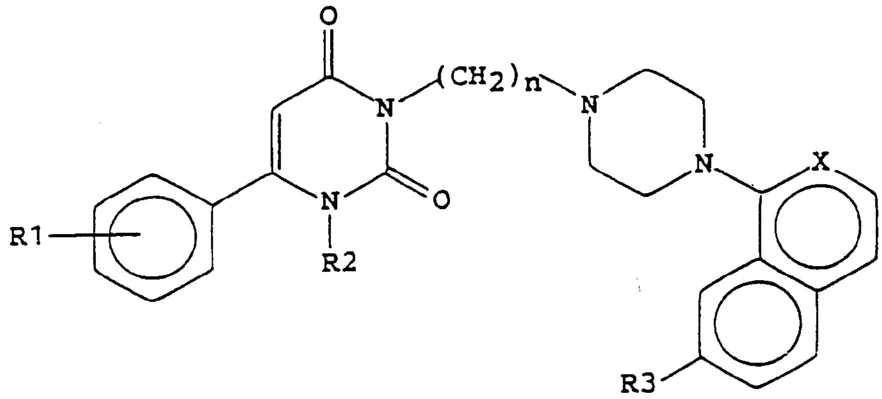

(I)

in der
R1 ein Wasserstoffatom, ein Halogenatom oder eine Methyl- oder Methoxygruppe,
R2 ein Wasserstoffatom, eine $C_1$-$C_4$-Alkylgruppe oder eine Benzylgruppe,
n eine ganze Zahl mit einem Wert von 2, 3 oder 4 und
X eine CH-Gruppe oder ein Stickstoffatom und
R3 ein Wasserstoffatom, ein Halogenatom oder eine Methoxygruppe, wenn X eine CH-Gruppe darstellt, oder lediglich ein Wasserstoffatom, wenn X ein Stickstoffatom darstellt, bedeuten sowie deren Additionssalze mit pharmakologisch annehmbaren Säuren.

2. Verfahren zur Herstellung der Verbindungen gemäß Anspruch 1, **dadurch gekennzeichnet**, daß man ein 6-Phenyl-1H,3H-pyrimidin-2,4-dion der allgemeinen Formel (II)

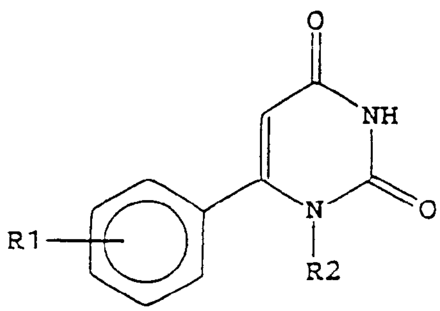

(II)

(in der R1 und R2 die in Anspruch 1 angegebenen Bedeutungen besitzen) mit einem Dihalogenderivat der allgemeinen Formel (III)

$Y(CH_2)_nCl$ (III)

(in der n die in Anspruch 1 angegebene Bedeutung besitzt und Y ein Brom- oder Chloratom darstellt), umsetzt und dann das in dieser Weise erhaltene Derivat der allgemeinen Formel (IV)

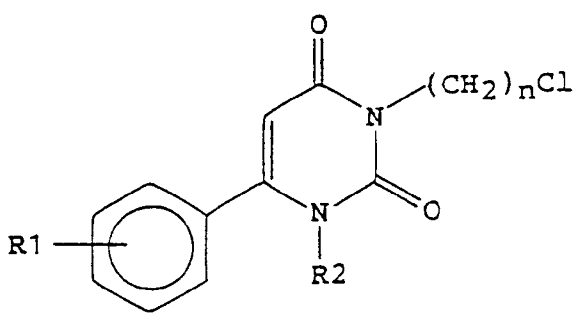

(IV)

mit einem Piperazinderivat der allgemeinen Formel (V)

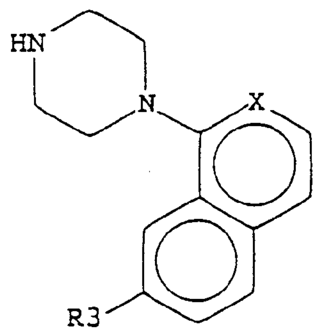

(V)

(in der X und R3 die in Anspruch 1 angegebenen Bedeutungen besitzen) umsetzt.

3. Verfahren zur Herstellung der Verbindungen nach Anspruch 1 der allgemeinen Formel (I), in der R2 ein Wasserstoffatom darstellt, **dadurch gekennzeichnet**,daß man ein 6-Phenyl-1H,3H-pyrimidin-2,4-dion der allbemeinen Formel (IIa, IIb)

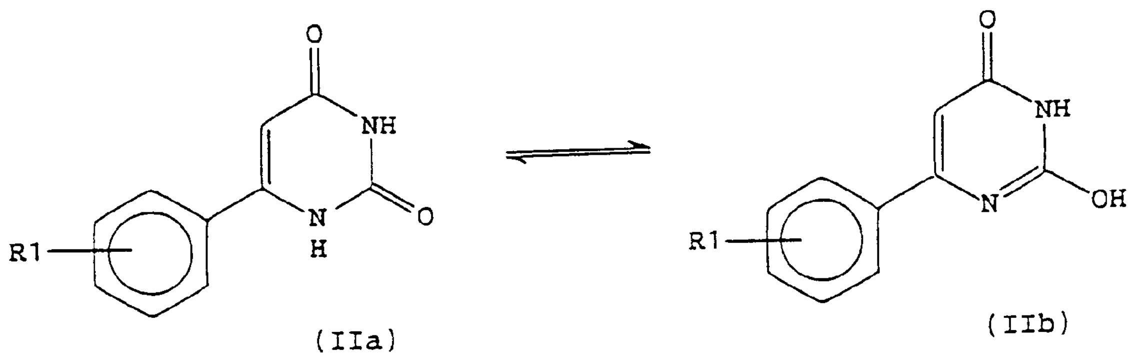

(IIa) (IIb)

(in der R1 die in Anspruch 1 angegebenen Bedeutungen besitzt) zunächst mit einem Alkalimetallhydrid und dann mit einem Dihalogenderivat der allgemeinen Formel (III) (in der n die in Anspruch 1 angegebene Bedeutung besitzt und Y ein Brom- oder Chloratom darstellt) umsetzt
und dann das in dieser Weise erhaltene bicyclische Derivat der allgemeinen Formel (IVa)

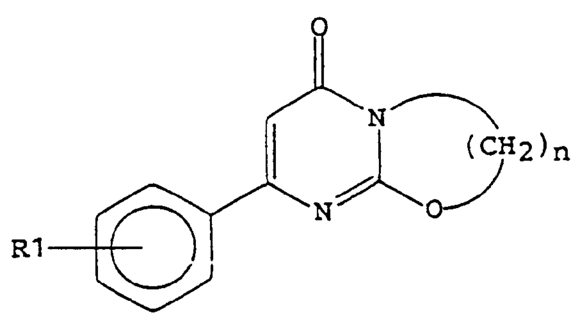

(IVa)

mit einem Piperazinderivat der allgemeinen Formel (V)

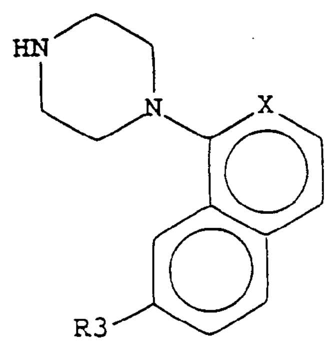

(V)

(in der X und R3 die in Anspruch 1 angegebenen Bedeutungen besitzen) umsetzt.

4. Pharmazeutische Zubereitung, **dadurch gekennzeichnet**, daß sie eine Verbindung gemäß Anspruch 1 in Kombination mit einem geeigneten Trägermaterial enthält.

5. 1-(Piperazin-1-yl)-isochinolin als Zwischenprodukt für das Verfahren nach Anspruch 2.

6. 1-(7-Fluor-naphth-1-yl)-piperazin als Zwischenprodukt für das Verfahren gemäß Anspruch 2.

**Patentansprüche für folgende Vertragsstaaten: ES, GR**

1. Verfahren zur Herstellung der Verbindungen der allgemeinen Formel (I)

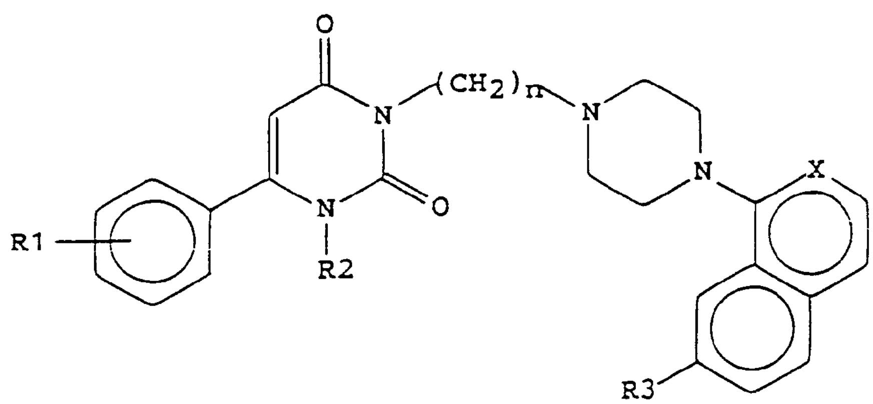

(I)

in der
R1 ein Wasserstoffatom, ein Halogenatom oder eine Methyl- oder Methoxygruppe,
R2 ein Wasserstoffatom, eine $C_1$-$C_4$-Alkylgruppe oder eine Benzylgruppe,
n eine ganze Zahl mit einem Wert von 2, 3 oder 4 und
X eine CH-Gruppe oder ein Stickstoffatom und
R3 ein Wasserstoffatom, ein Halogenatom oder eine Methoxygruppe, wenn X eine CH-Gruppe darstellt, oder lediglich ein Wasserstoffatom, wenn X ein Stickstoffatom darstellt, bedeuten,
**dadurch gekennzeichnet**, daß man ein 6-Phenyl-1H,3H-pyrimidin-2,4-dion der allgemeinen Formel (II)

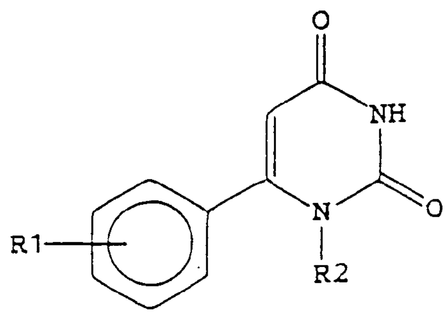

(II)

(in der R1 und R2 die oben angegebenen Bedeutungen besitzen) mit einem Dihalogenderivat der allgemeinen Formel (III)

$Y(CH_2)_nCl$ (III)

(in der n die oben angegebene Bedeutung besitzt und Y ein Brom- oder Chloratom darstellt), umsetzt und dann das in dieser Weise erhaltene Derivat der allgemeinen Formel (IV)

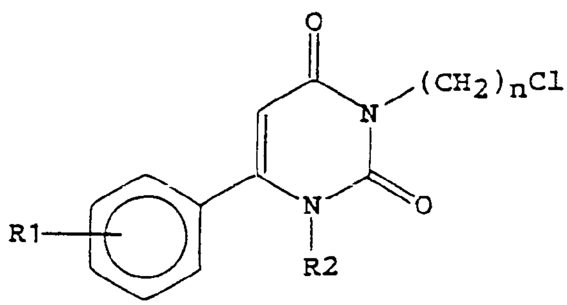

(IV)

mit einem Piperazinderivat der allgemeinen Formel (V)

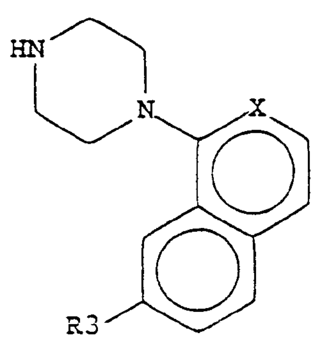

(V)

(in der X und R3 die oben angegebenen Bedeutungen besitzen) umsetzt.

2. Verfahren zur Herstellung der in Anspruch 1 definierten Verbindungen der allgemeinen Formel (I), in der R2 ein Wasserstoffatom bedeutet, **dadurch gekennzeichnet**, daß man ein 6-Phenyl-1H,3H-pyrimidin-2,4-dion der allgemeinen Formel (IIa, IIb)

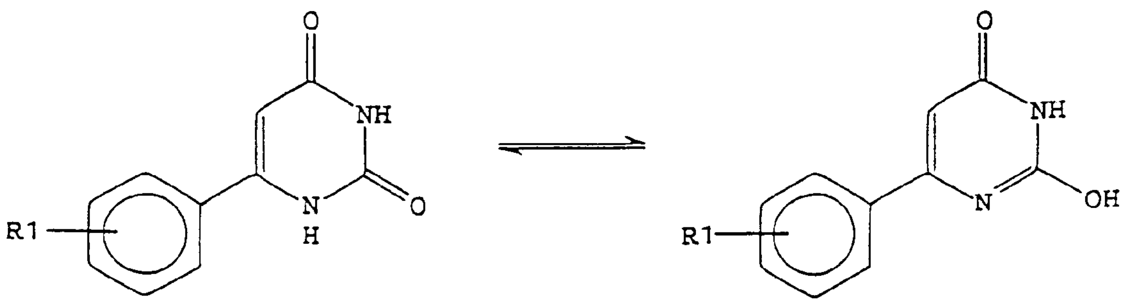

(IIa) (IIb)

(in der R1 die in Anspruch 1 angegebenen Bedeutungen besitzt) zunächst mit einem Alkalimetallhydrid und dann mit einem Dihalogenderivat der allgemeinen Formel (III) (in der n die in Anspruch 1 angegebene Bedeutung besitzt und Y ein Brom- oder Chloratom darstellt) umsetzt
und dann das in dieser Weise erhaltene bicyclische Derivat der allgemeinen Formel (IVa)

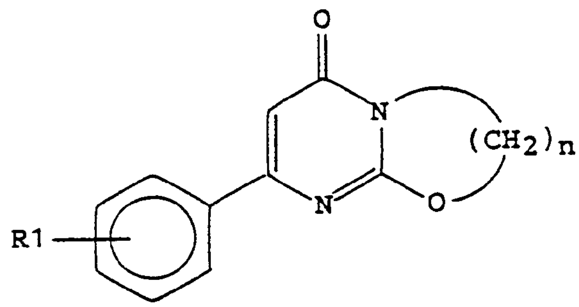

(IVa)

mit einem Piperazinderivat der allgemeinen Formel (V)

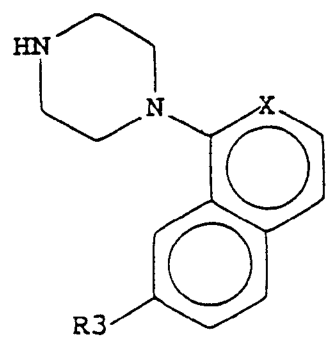

(V)

(in der X und R3 die in Anspruch 1 angegebenen Bedeutungen besitzen) umsetzt.

## Claims

### Claims for the following Contracting States : AT, BE, CH, DE, FR, GB, IT, LI, LU, NL, SE

**1.** Compounds corresponding to the general formula (I)

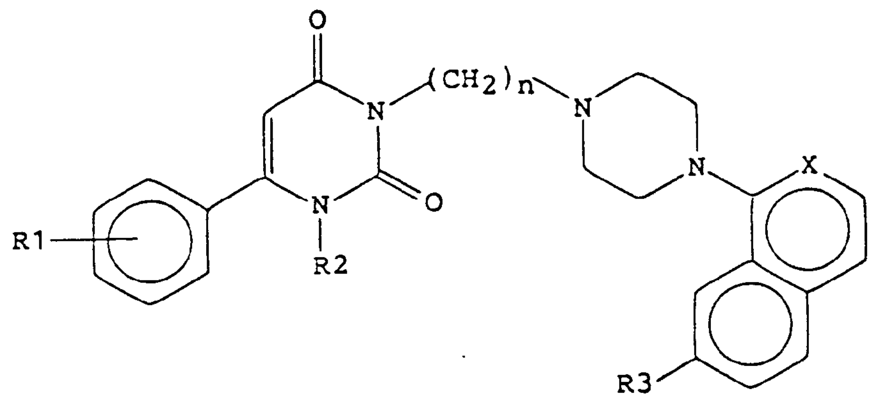

(I)

in which
R1 denotes a hydrogen or halogen atom or a methyl or methoxy group,
R2 denotes a hydrogen atom, a $C_1$-$C_4$ alkyl group or a benzyl group,
n denotes an integer equal to 2, 3 or 4,
X denotes a CH group or a nitrogen atom, and
R3 denotes a hydrogen or halogen atom or a methoxy group when X denotes a CH group, or only a hydrogen atom when X denotes a nitrogen atom,
as well as their addition salts with pharmacologically acceptable acids.

2. Process for preparing the compounds according to Claim 1, characterised in that a 6-phenyl-2,4(1*H*,3*H*)pyrimidinedione of general formula (II)

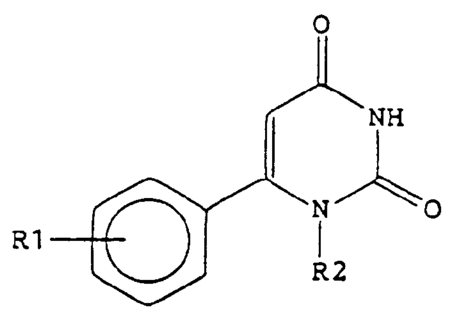

(II)

(in which R1 and R2 are as defined in Claim 1)
is reacted with a dihalo derivative of general formula (III)

$Y(CH_2)_nCl$ (III)

(in which n is as defined in Claim 1 and Y denotes a bromine or chlorine atom),
and the derivative of general formula (IV) thereby obtained

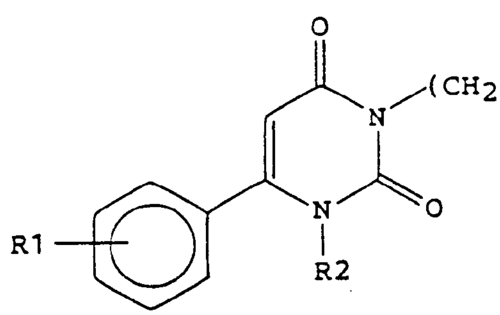

is then reacted with a piperazine derivative of general formula (V)

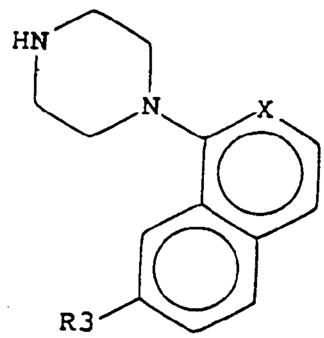

(V)

(in which X and R3 are as defined in Claim 1).

**3.** Process for preparing the compounds according to Claim 1, in the general formula (I) of which R2 denotes a hydrogen atom, which process is characterised in that the 6-phenyl-2,4(1*H*,3*H*)-pyrimidinedione of general formula (IIa, IIb)

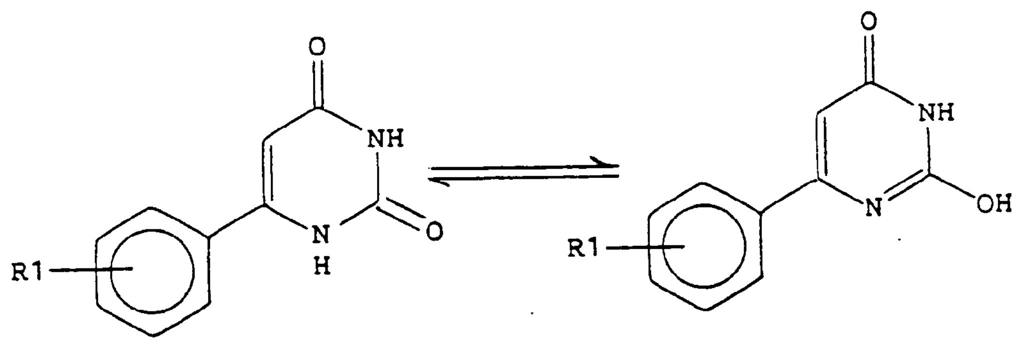

(IIa) (IIb)

(in which R1 is as defined in Claim 1)
is reacted first with an alkali metal hydride and then with a dihalo derivative of general formula (III) (in which n is as defined in Claim 1 and Y denotes a bromine or chlorine atom),
and the bicyclic derivative of general formula (IVa) thereby obtained

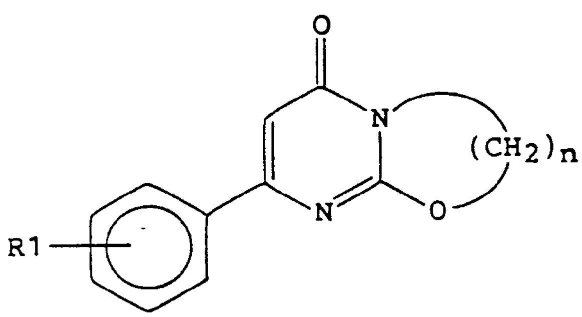

(IVa)

is then reacted with a piperazine derivative of general formula (V)

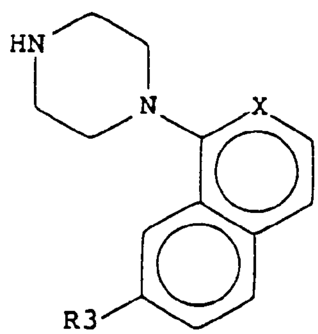

(V)

(in which X and R3 are as defined in Claim 1).

4. Pharmaceutical composition, characterised in that it contains a compound according to Claim 1, in combination with a suitable excipient.

5. 1-(1-Piperazinyl)isoquinoline, as a compound necessary as an intermediate in the process according to Claim 2.

6. 1-(7-Fluoro-1-naphthyl)piperazine, as a compound necessary as an intermediate in the process according to Claim 2.

**Claims for the following Contracting States : ES, GR**

1. Process for preparing compounds corresponding to the general formula (I)

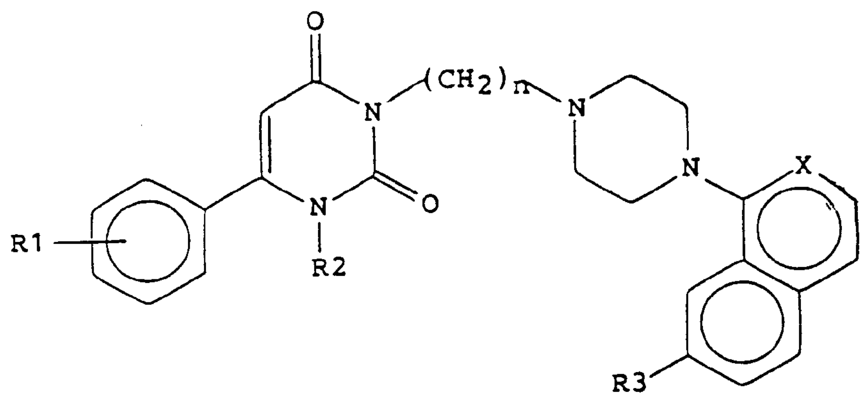

(I)

in which
R1 denotes a hydrogen or halogen atom or a methyl or methoxy group,
R2 denotes a hydrogen atom, a $C_1$-$C_4$ alkyl group or a benzyl group,
n denotes an integer equal to 2, 3 or 4,
X denotes a CH group or a nitrogen atom, and
R3 denotes a hydrogen or halogen atom or a methoxy group when X denotes a CH group, or only a hydrogen atom when X denotes a nitrogen atom,
which process is characterised in that a 6-phenyl-2,4-(1*H*,3*H*)-pyrimidinedione of general formula (II)

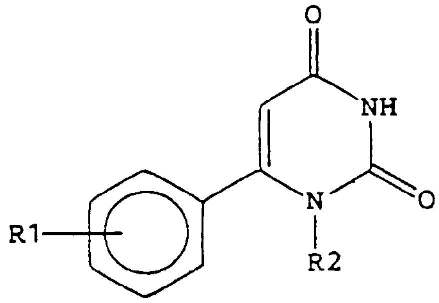

(II)

(in which R1 and R2 are as defined above)
is reacted with a dihalo derivative of general formula (III)

$Y(CH_2)_nCl$ (III)

(in which n is as defined above and Y denotes a bromine or chlorine atom), and the derivative of general formula (IV) thereby obtained

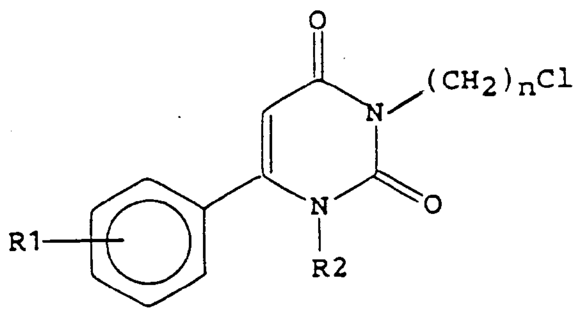

(IV)

is then reacted with a piperazine derivative of general formula (V)

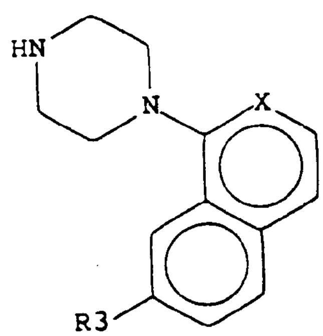

(V)

(in which X and R3 are as defined above).

2. Process for preparing the compounds defined in Claim 1, and in the general formula (I) of which R2 denotes a hydrogen atom, which process is characterised in that the 6-phenyl-2,4(1*H*,3*H*)-pyrimidinedione of general formula (IIa, IIb)

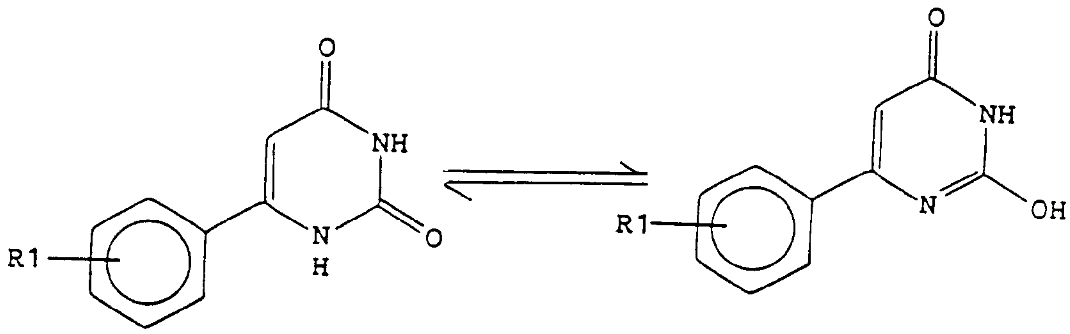

(IIa) (IIb)

(in which R1 is as defined in Claim 1) is reacted first with an alkali metal hydride and then with a dihalo derivative of general formula (III) (in which n is as defined in Claim 1 and Y denotes a bromine or chlorine atom), and the bicyclic derivative of general formula (IVa) thereby obtained

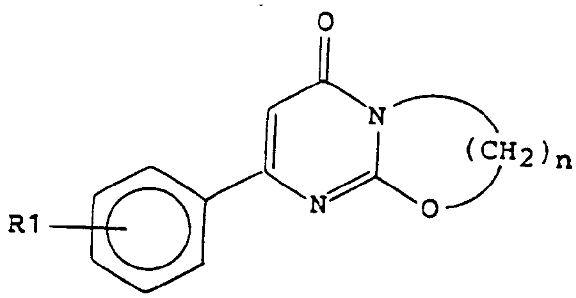

(IVa)

is then reacted with a piperazine derivative of general formula (V)

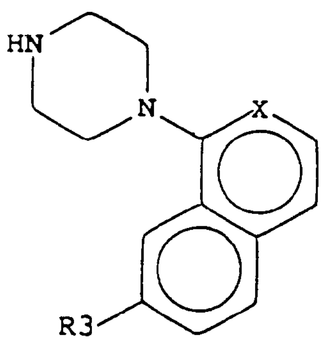

(V)

(in which X and R3 are as defined in Claim 1).